Europäisches Patentamt

(19) European Patent Office     (11) Publication number: **0 244 446**
Office européen des brevets                         **B1**

(12)        **EUROPEAN PATENT SPECIFICATION**

(45) Date of publication of patent specification: **22.08.90**     (51) Int. Cl.⁵: **A 61 N 1/36**

(21) Application number: **86906215.8**

(22) Date of filing: **06.10.86**

(86) International application number:
**PCT/US86/02114**

(87) International publication number:
**WO 87/01947 09.04.87 Gazette 87/08**

(54) **MYOCARDIAL CONTRACTILITY-SENSITIVE PACER.**

(30) Priority: **07.10.85 US 785138**

(43) Date of publication of application:
**11.11.87 Bulletin 87/46**

(45) Publication of the grant of the patent:
**22.08.90 Bulletin 90/34**

(84) Designated Contracting States:
**AT BE CH DE FR GB IT LI LU NL SE**

(56) References cited:
**EP-A-0 080 347**
**EP-A-0 178 528**
**FR-A-2 403 775**
**US-A-3 857 399**
**US-A-4 026 303**
**US-A-4 052 991**
**US-A-4 424 812**
**US-A-4 543 954**
**US-A-4 600 017**

(73) Proprietor: **THOMAS JEFFERSON UNIVERSITY**
**11th and Walnut Streets**
**Philadelphia Pennsylvania19107 (US)**

(72) Inventor: **KRESH, Yasha, J.**
**266 W. Rittenhouse Square**
**Philadelphia, PA 19103 (US)**

(74) Representative: **Baillie, Iain Cameron et al**
**c/o Ladas & Parry Isartorplatz 5**
**D-8000 München 2 (DE)**

Courier Press, Leamington Spa, England.

## Description

Background of the Invention

This invention relates to demand-type pacing systems and, more particularly, to rate-adaptive pacing systems for controlling cardiac pacing rate as a function of a monitored cardiac parameter such as myocardial viability.

Rate adaptive pacemaker systems are known in the art and are receiving increasing attention and commercialization. Historically, the first rate adaptive pacing systems have been the dual chamber systems, e.g. atrial synchronous systems, where the pacing of the ventricle is coupled to or synchronized with detected atrial beats. Such dual chamber systems have the clear advantage of optimally simulating normal physiological response, since they derive the timing of the pacing pulses from the atrial response which is induced by the natural heart pacemaker. However, dual chamber systems have certain disadvantages which have been recognized in the literature, as well as certain practical disadvantages. It has been reported that atrial synchronous pacing is not suitable for up to 76% of pacemaker-indicated patients. See Rate Responsive Pacing, Rickards et al, Clin. Prog. Pacing and Electrophysiology, Vol. 1, No. 1, 1983, pp. 12-18.

Dual chamber pacemakers have never been prescribed with the frequency of single chamber pacemakers, for the practical reasons of expense, the need to place pacing leads into both chambers and the potential complexities of dual chamber pacing (such as pacemaker mediated tachycardia) which can be avoided in patients who can be well treated with the simpler single chamber systems.

The single chamber demand pacemaker, while simplest and most reliable in terms of system complexity, carries the fundamental limitation of not being able to adapt its response to normal patient needs. It is well known that the requirements of blood flow vary widely as a function of both physiological (exercise) and emotional occurrences. The normal heart adjusts blood flow by varying both stroke volume and rate, the blood flow being the result of the product of those two variables. While a single chamber pacemaker cannot adjust stroke volume, it can adjust heart rate by the relatively simple technical expedient of adjusting the rate of the delivered pulses. The adjustment of rate alone is considered to provide about 80% of the total blood flow change. See the above-cited Rickards et al paper.

U.S. Patent 4,052,991 discloses a pacemaker which may include an intramyocardial pressure sensor positioned on the lead, for generating signals representative of intramyocardial pressure (IMP), and for changing the base timing interval of the pacing pulses as a function of sensed IMP. However, U.S. Patent 4,052,991 teaches measuring IMP during the time of rest of the cardiac muscle, and using only very slow variations of the IMP measurement.

In response to the recognized desirability of being able to automatically adapt pacing rate, e.g., increase exercise tolerance, there has been a great deal of work and progress, including several commercial pacemakers incorporating different feedback systems for controlling pacing rate as a function of one or more sensed cardiac or body parameters. For a summary of such systems, see "Principles of Exercise-Responsive Pacemakers," IEEE Engineering in Medicine and Biology Magazine, June 1984, pp. 25-29; "The Exercise-Responsive Cardiac Pacemaker," IEEE Transaction on Biomedical Engineering, Vol. BME 31, No. 12, December 1984.

The system of this invention is based upon our observation that the rate adaptive pacemaker principle is efficiently carried out by controlling the function of one or more parameters which normally and directly control (or are concomitant with) pacing rate in a healthy person. This approach is contrasted to many of the systems that are presently being investigated, wherein indirect parameters such as respiration rate, oxygen saturation, etc. are monitored and utilized in the rate adaptive feedback loop. It is known that there are a number of pathways that directly affect the heart, one being the cardiac sympathetic nerves, and the other being direct release of hormones or catecholamines into the bloodstream. In a normal state the neuroendocrine system stimulates various cardiovascular receptor sites, thus controlling both the heart rate, which is triggered by the natural pacemaker (sinus node) and myocardial contractility which brings about an increase in hemodynamics (pressure-flow). In patients who do not have an intact conduction system, e.g., sino-atrial node dysfunction, the concomitant events that should take place, namely increase in rate and contractility, are disrupted or impaired. Thus we make our observation that the ability to directly detect changes in sympathetic drive, ventricular contractility or circulation catecholamine levels constitute an appropriate means of obtaining information for control of a pacemaker.

It is very unlikely that an artificial chemoreceptor for detecting sympathetic tone or catecholamine level can be perfected in the near future, although such an accomplishment could provide a medically attractively solution to pacemaker control. Another more practical approach for the present in achieving a closed-loop physiologically responsive pacing system is to measure changes in the contractile state of the cardiac muscle itself. This can be accomplished, at least presently, by two different means. A first means is to measure stress in the muscle wall (intramyocardial pressure, or IMP) and the other is to measure the regional wall thickening or fiber-shortening of the muscle. The former can be done by use of ultrasonic A-scan imaging, and the latter with pressure, and pacing rate is varied directly as a function of the sensed IMP signal characteristics. The high sensitivity of the sensor provides system reliability, and an overall system response of less than 30 seconds is achieved.

Brief Description of the Drawings

Fig. 1A is a schematic illustration of a lead tip placed in the myocardium of the left ventricle, the lead tip containing an IMP sensor as well as pacing electrodes.

Figs. 1B and 1C are schematic illustrations of an electrode tip showing a pressure sensor and electrodes.

Fig. 1D is a schematic illustration of an alternate electrode tip placed in the myocardium.

Fig. 2A is a schematic illustration showing a screw in tip placed in the myocardium of the left ventricle, and a transvenous catheter with another tip positioned in the myocardium at the septum or apex of the right ventricle.

Fig. 2B is an enlarged schematic showing the detail of the tip of the transvenous catheter illustrated in Fig. 2A.

Fig. 3 is a block diagram showing the overall pacing system of this invention.

Figs 4A and 4B show an illustrative algorithm for use in the pacemaker of this invention.

Fig. 4C illustrates changing heart rate as a function of IMP and the derived IMP function FNC(t).

A Description of the Preferred Embodiments

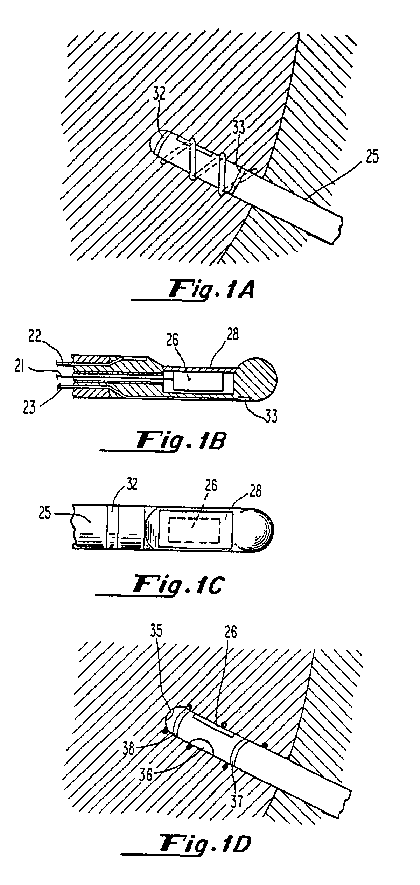

As discussed above, and illustrated in the drawings, this invention comprises a pacemaker system for controlling pacing rate as a function of sensed myocardial contractility. The embodiments utilize a pressure sensor for sensing of IMP. Referring to Fig. 1A, there is shown a schematic of a sensor adapted to be placed in the myocardium of the left ventricle. The sensor preferably has threads — enabling it to be screwed into the myocardium. The sensor 26 is positioned on the tip of lead 25, which lead also carries electrodes 32, 33. The pressure sensor and electrodes are connected by suitable conductors, not shown, to the pacemaker of the system. As seen in Figs. 1B and 1C, the pressure sensor is implanted within the tip of the lead, and may be covered by a silicone rubber diaphragm 28 or other suitable cover which permits the external pressure to be communicated through to the sensor. The sensor is shown communicating through the lead by means of one or more conductors 21. The electrodes 32, 33, communicate electrically through the lead by conductors 22, 23. As is well known, the pacing system may be unipolar or bipolar, this being a matter of design choice.

A preferred sensor for assessing IMP is a MIKRO-TIP (registered trademark) pressure sensor made by Melar Instruments, or an MMI-Gaeltec transducer. The pressure sensor is preferably mounted at the end of a 3-Fr catheter, being 1 mm in diameter. The transducer operates on the principle of producing a varying electrical signal output proportional to the magnitude of sensed pressure stress resulting from deformation or strain of a diffused semi-conductor. Conventional electronics (full-bridge circuit) converts this resistance change to voltage change indicative of stress or pressure applied directly on the sensing area.

Fig. 1D is another schematic view of a catheter or lead tip for use in this invention, which includes one or more additional sensors as well as the IMP sensor. A thermistor 35 is illustrated at the very end of the tip, and a pH sensor is also illustrated at 36. Appropriate leads through the catheter length are utilized for communicating the sensor signals back to the pacemaker. As discussed hereinbelow, the utilization of additional sensed parameters is possible, the additional information being utilized to correlate the IMP data in determining the desired pacing rate. Also, as mentioned above, although not illustrated, additional types of myocardial contraction sensors are available, i.e. ultrasonic A-scan imaging and intramural.sonomicrometric sensors.

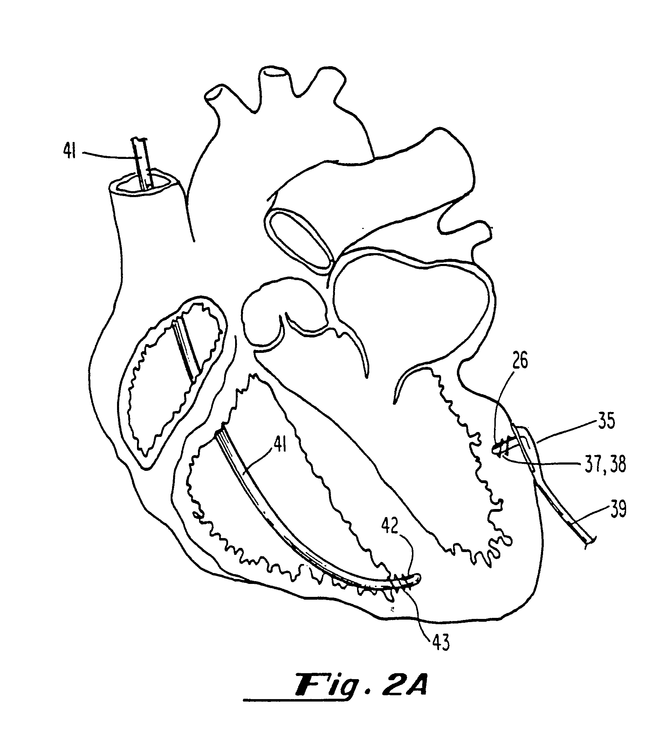

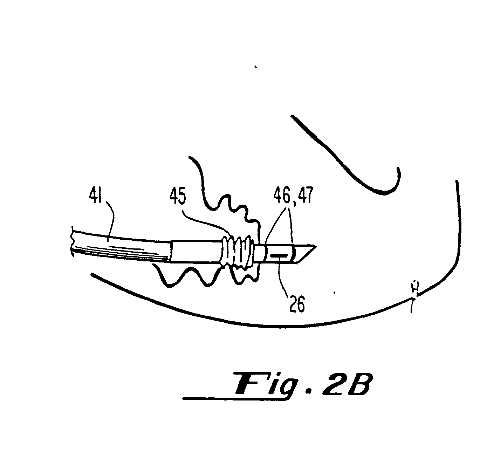

Referring now to Fig. 2A, there is shown a representation of a human heart with two illustrations of lead placements. A screw-in epicardial-type lead 39 having a tip head 35 is shown screwed into the heart wall, penetrating through the epicardium into the myocardium of the left ventricle. The technique that is used with this type of electrode is the known technique of securing the electrode to the heart wall through a left thoracotomy. An alternate embodiment uses a conventional transvenous catheter, or lead system as illustrated by lead 41, having the pressure sensor located as indicated at 42 at the distal tip end, and having fixation means 43. It is known in the art that transvenous electrodes are available for insertion into the right ventricle, with varying types of fixation elements for fixing the lead against and indeed into the heart wall. As indicated in Fig. 2B, the transvenous electrode lead 41 may have a retractable sleeve 45 operable in a known fashion for exposing the far distant tip of the lead after initial placement within the right ventricle. For placement in the right ventricle, it is noted that the right ventricle wall is relatively thin and thus not preferable for providing a good indication for wall stress changes resulting from changing contractility. It is accordingly necessary to position or steer the lead so that it is anchored in the apex, or the septal wall.

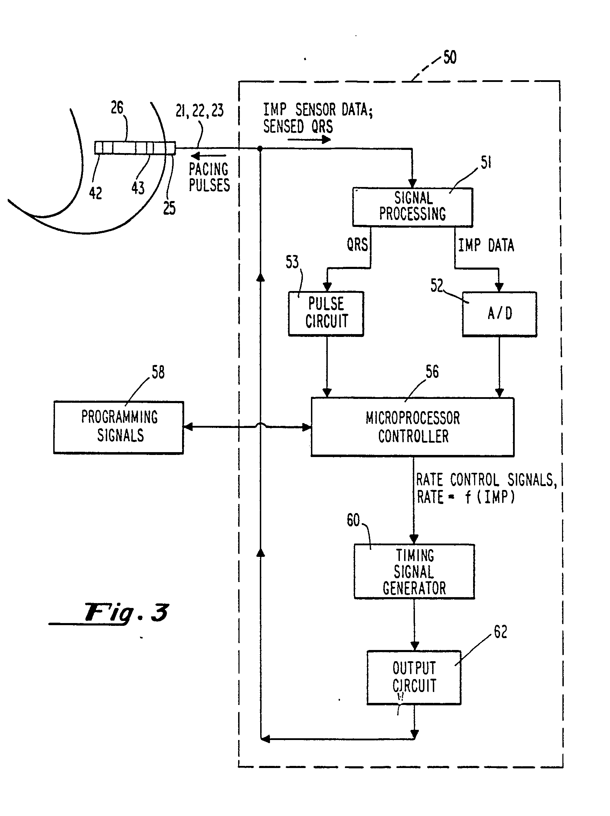

Referring now to Figure 3 there is shown a schematic diagram illustrative of the pacing system of this invention. As illustrated, a pacemaker 50, suitably implanted in the patient, communicates with the lead tip 25 which is fixed in the myocardium, through electrical conductors 21, 22, 23. Pacemaker 50 is suitably a programmable pacemaker and can receive programming signals from an external source 58 of known design, and additionally can communicate information back out to the external apparatus 58. As illustrated, the pacemaker is preferably microprocessor controlled in order to provide optimum control, as known in the art. Suitable battery source means, not illustrated, provide power to the pacemaker.

Two inputs are received from the implanted lead tip, namely the IMP sensor data and the sensed QRS signal. These are inputted to signal processing circuit 51 of conventional design, which detects the inputs and provides clear signal outputs. For the IMP data, signal processing circuit 51 may suitably include a bridge-type configuration for measuring the impedance change of pressure sensor 26. The IMP data is outputted to an analog to digital converter 52, which converts the analog signal representative of IMP into a digital format suitable for subsequent digital processing. The detected analog QRS signal is inputted to pulse circuit 53, which provides a timing pulse output indicative of the occurrence of the QRS signal when a natural heart beat has indeed been detected. The output of pulse circuit 53 is likewise inputted to microprocessor control circuit 56.

Microprocessor controller 56 comprises the means, in hardware and/or software form, for carrying out the well known control functions performed in a modern demand pacemaker. Thus, the controller circuit 56 comprises a timing circuit for timing out a normal pulse stimulus rate, resetting the timing means upon the occurrence of a QRS, receiving programming signals from external apparatus 58, sensing battery life, etc. Controller 56 also preferably utilizes a microprocessor and associated memory which acts in cooperation with the required analog circuitry for performing controller functions. A microprocessor is preferred for carrying out the algorithm which is used for determining pacing rate as a function of sensed IMP. The algorithm may be software-controlled by instructions stored in RAM or ROM, or it may be carried out by conventional hardwired logic circuitry. In the preferred embodiment, the microprocessor and a portion of associated memory comprise logic means for carrying out the desired algorithm, e.g., a threshold seeking algorithm using a non-linear average process. Further, the associated memory may be re-programmed to change the algorithm, either by changing the mathematical step performed, or by changing constants which are used in calculating the relationship between sensed IMP and desired pacing rate. Further, the algorithm may use other sensed parameters, such as a detected pH or temperature in the heart muscle for modifying the rate determination. The output of the controller 56 containing information representative of the desired rate and the timing of pulse signals is connected to timing signal generator 60. Generator 60 produces a timing signal in response to the controller information, which timing signal is connected to output circuit 62. Output 62 responds to the timing signals by producing output pacing pulses of desired energy and form, which are connected to the pacing lead and hence to the electrodes 42, 43 at the lead tip.

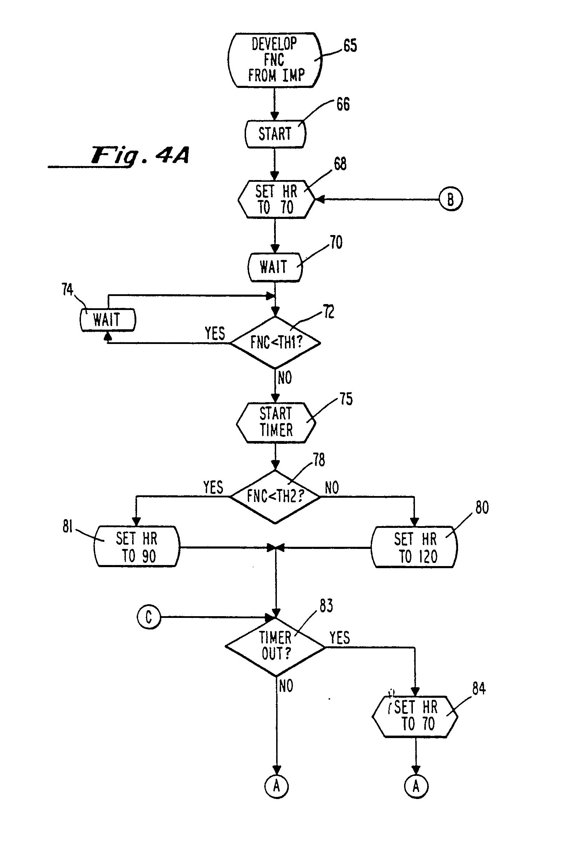

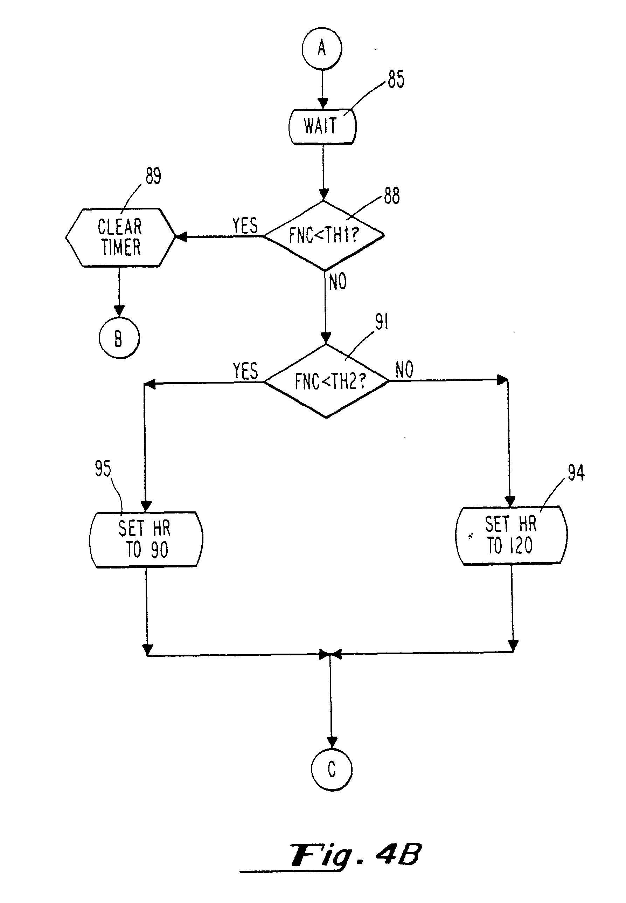

Referring now to Figs. 4A-4C, there is illustrated an algorithm for setting heart rate (HR) as a function of determined IMP. The preferred embodiment is illustrated as a software flowchart for use by a suitable microprocessor, as discussed above in connection with block 56 of Fig. 3. The system preferably incorporates a non-linear signal averaging means for processing the IMP signal. Specifically, a median average is obtained, followed by non-linear signal averaging. All data points around the median value and falling within a prescribed range are averaged together. This process results in relative noise immunity and prevents false trigger rates.

The algorithm continuously develops an IMP function FNC, as shown at 65. This algorithm may be operated, for example, every pacer cycle or after an accumulation of IMP data over a predetermined number of cycles. The IMP function FNC is preferably characterized by a regression analysis between the heart rate and intramyocardial pressure using both the peak value of IMP and the peak slope as parameters for controlling the heart rate and determining threshold levels (e.g., TH 1 and TH 2). FNC may also be made a function of one or more additional parameters measured by the lead or another sensor.

The algorithm for setting heart rate is started as indicated at 66. The algorithm may be started, or initiated, automatically during any pacer cycle, or it may be started only upon indication of change in IMP or FNC. The heart rate is initially set to 70 beats/minute (or any other desired basal rate) as seen at 68. The pacer then waits, at 70, for a convenient portion of the next pacer cycle. The function FNC(t) is compared to a preset threshold value TH1 at 72. This threshold value can be programmed externally to accommodate particular patient parameters. If the resultant function FNC(t) is less than the given threshold, the pacemaker remains at the preset heart rate, and waits at 74 for the next pacer cycle. If this condition is not satisfied, a timer is begun at 75. This timer can be programmed externally such that it does not allow the elevated heart rate to be maintained for more than 30 minutes or other desired time-interval. This represents a safety feature preventing a sustained high rate for periods well above a normal physiological response to stress.

The sequence continues at 78 where FNC is compared to another threshold level, identified as TH2. When this threshold level is satisfied, the algorithm branches at 80 where the heart rate is set to 120 beats/minute (which is a typical upper limit). If the given function is less than the TH2 level, the program branches at 81 where the heart rate is set at 90 (corresponding to the first level of heart rate change). Following this, at 83 the timer is checked, and if the given period has elapsed, the program goes to 84 and the pacemaker reverts automatically to the basal rate (in this particular example it is 70 beats/minute). If the time interval is not exhausted, the program continues directly to block 85 and waits for the next cycle. At 88, another check is made to see if the given function (FNC) is less than the first threshold level (TH1). If this condition is satisfied, the timer is cleared at 89 and the program returns to 68 where heart rate is set to 70. If the given function is not less than TH1, the program proceeds at 91 and goes through another check to see if FNC is less than TH2. If the resultant outcome is negative, heart rate is set to 120 at block 94; if the outcome state is positive, heart rate is set at 90 at block 95. At both blocks 94 and 95, the status of the current heart rate is checked and, if already at the indicated rate, the pacemaker is prevented from resetting. The program then goes back to 83 where the timer is checked, and the cycle is repeated again after waiting at 85.

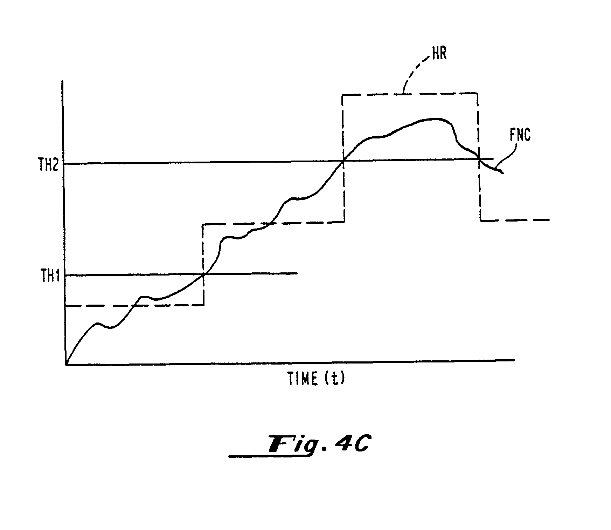

Figure 4C illustrates changes with time of function FNC(t), the two preset threshold levels, and the corresponding heart rate response shown as a dashed line. The algorithm is not restricted to only two rates beyond the basal rate, but in fact can be made to respond to multiple discrete rates or monotonically to the IMP-function changes with time. In addition, it is possible to telemeter all of the measured values to external apparatus to allow optimal programmability of IMP-function parameters and guide sensor for maximal IMP-signal recording.

**Claims**

1. A rate adaptive pacemaker system including pulse means for generating pacing pulses, timing means (56, 60, 62) for generating timing signals controlling the time and rate of said pacing pulses, and connected to deliver said timing signals to said pulse means so as to control the rate of generated pacing pulses, a lead (25) for delivering said pacing pulses to the heart of said patient, said lead (25) being connected to said pulse means and having electrode means (32, 33) for delivering said pacing pulses to said patient heart, said lead means also having a sensor (26) for sensing patient heart beats and for generating heart signals representative thereof, a pressure sensor positioned on the lead (25) for implantation in the myocardium to sense myocardial pressure and generate a signal representative thereof, and signal processing means (51) connected to said lead to receive said intramyocardial pressure signals and for processing same, and a controller (56) connected to said signal processing means (51) to receive the processed intra-myocardial pressure signal and having an output connected to said timing means for controlling the rate of generation of pacing pulses in accordance with sensed intramyocardial pressure, characterized in that the controller is constructed to respond to control the pulsing rate in proportion to a combination of the peak value of intramyocardial pressure and the peak rate of change of intramyocardial pressure during contraction such that it derives from the measured intramyocardial pressure an indication of myocardial contractility and controls the rate of generation of pulses in accordance therewith.

2. The pacemaker system as claimed in claim 1, characterized in that said controller includes a microprocessor (56) for receiving said signals from said signal processing means (51) and for carrying out an algorithm for determining said rate as a function of said received signals.

3. A pacemaker system as claimed in claim 2, characterized in that said signal processing means (51) comprises analog to digital converting means for converting said intramyocardial pressure signals into digital signals for subsequent use by said controller.

4. A pacemaker system as claimed in any one of the preceding claims, characterized in that said controller (56) is programmable by an external source (58) of programming signals.

5. A pacemaker system as claimed in any one of the preceding claims, characterized in that the lead (25) has a distal tip, said intramyocardial pressure sensor and said electrode means being positioned in close proximity to said distal tip.

6. The pacemaker system as claimed in claim 5, characterized in that the signal processing means (51) is operable to receive and process said intramyocardial pressure signals on a cycle to cycle basis.

7. A pacemaker system as claimed in any one of the preceding claims, characterized in that said lead comprises a screw-in fixation tip (35) with an intramyocardial pressure sensor (42) mounted thereon for engaging the heart myocardium when the tip is screwed into the myocardium.

8. The pacemaker system as claimed in any one of the preceding claims, characterized in that said lead includes fixation means for fixing said intramyocardial pressure sensor in the heart myocardium.

9. A pacemaker system as claimed in any one of the preceding claims, characterized in that an additional sensor is located proximate the tip of the lead for sensing at least one additional parameter associated with said patient's heart and for generating additional parameter signals representative of said additional parameter, said signal processing means being connected to receive and process said additional signals, and in that said controller means determines said pacing rate as a function of said additional parameter.

10. A pacemaker system as claimed in any one of the preceding claims, characterized in that the controller includes storage means for holding algorithm instructions for determining pacing rate as a function of patient intramyocardial pressure.

11. A pacemaker system as claimed in claim 10, characterized by external programming apparatus for inputting algorithm instructions into said controller means.

12. A pacemaker system as claimed in claim 11, characterized in that the external programming apparatus has means for inputting data into the controller to cause said controller to control pacing rate as a function of at least one other parameter.

13. A pacemaker system as claimed in any of claims 1 to 12 characterized in that the controller has threshold means for comparing said contractility signals with at least one predetermined threshold during each cycle of a plurality of continuous pacing cycles, and in that said controller determines pacing rate as a function of said comparing.

**Patentansprüche**

1. Frequenzveränderliches Herzschrittmachersystem mit

einem Impulsegeber zum Erzeugen von Herzschrittmacherimpulsen;

einem Zeitgeber (56, 60, 62) der zum Erzeugen von Zeitsteuersignalen zur Steuerung der Zeit und der Frequenz der Herzschrittmacherimpulse dient und so geschaltet ist, daß er die Zeitsteuersignale an den Impulsgeber abgibt, um die Frequenz der erzeugten Herzschrittmacherimpulse zu steuern;

einer Zuleitung (25), die zur Abgabe der Herzschrittmacherimpulse an das Herz des Patienten dient und mit dem Impulsgeber verbunden ist und

Electrodenmittel (32, 33) zur Abgabe der Herzschrittmacherimpulse an das Herz des Patienten aufweist,

wobei die Zuleitung ferner einen Sensor (26) aufweist, der dazu dient, die Herzschläge des Patienten zu erfassen und diese darstellende Herzsignale zu erzeugen;

einem an der Zuleitung (25) angeordneten Drucksensor, der dazu bestimmt ist, in dem Herzmuskel implantiert zu werden, um den Herzmuskeldruck zu erfassen und ein diesen darstellendes Signal zu erzeugen;

einer mit der Zuleitung verbundenen Signalverarbeitungseinrichtung (51) zum Empfangen und Verarbeiten der Herzmuskelinnendrucksignale;

und mit einem Regler (56), der mit der Signalverarbeitungseinrichtung (51) verbunden ist, um das verarbeitete Herzmuskelinnendrucksignal zu empfangen, und der

einen mit dem Zeitgeber verbundenen Ausgang hat, der in Abhängigkeit von dem erfaßten Herzmuskelinnendruck die Frequenz der Erzeugung der Herzschrittmacherimpulse steuert, dadurch gekennzeichnet, daß der Regler so ausgebildet ist, daß durch seine Ansprache die Impulsfrequenz proportional der Kombination des Spitzenwertes des Herzmuskelinnendruckes und des Spitzenwertes des Geschwindigkeit der Veränderung des Herzmuskelinnendruckes während der Kontraktion gesteuert wird, so daß der Regler von dem gemessenen Herzmuskelinnendruck eine Anzeige der Kontraktilität des Herzmuskels ableitet und in Abhängigkeit davon die Frequenz der Impulserzeugung steuert.

2. Herzschrittmachersystem nach Anspruch 1, dadurch gekennzeichnet, daß der Pegler einen Mikroprozessor (56) besitzt, der dazu dient, die genannten Signale von der Signalverarbeitungseinrichtung (51) zu empfangen und einen Algorithmus zur Bestimmung der Frequenz als Funktion der empfangenen Signale durchzuführen.

3. Herzschrittmachersystem nach Anspruch 2, dadurch gekennzeichnet, daß die Signalverarbeitungseinrichtung (51) einen Analog-digital-Umsetzer aufweist, der dazu dient, die Herzmuskelinnendrucksignale in digitale Signale für die darauf-folgende Verwendung in dem Regler umzusetzen.

4. Herzschrittmachersystem nach einem der vorhergehenden Ansprüche, dadurch gekennzeichnet, daß der Regler (56) von einer externen Quelle (58) von Programmiersignalen programmierbar ist.

5. Herzschrittmachersystem nach einem der vorhergehenden Ansprüche, dadurch gekennzeichnet, daß die Zuleitung (25) eine distale Spitze hat und daß der Herzmuskelinnendrucksensor und die Elektrodenmittel in nächster Nähe der distalen spitze angeordnet sind.

6. Herzschrittmachersystem nach Anspruch 5, dadurch gekennzeichnet, daß die Signalverarbeitungseinrichtung (51) derart betätigbar ist, daü sie die Herzmuskelinnendrucksignale Zyklus für Zyklus empfängt und verarbeitet.

7. Herzschrittmachersystem nach einem der vorhergehenden Ansprüche, dadurch gekennzeichnet, daß die Zuleitung eine einschraubbare Befestigungsspitze (35) besitzt, auf der ein Herzmuskelinnendrucksensor (42) montiert ist, der bei in den Herzmuskel eingeschraubter Spitze an dem Herzmuskel angreift.

8. Herzschrittmachersystem nach einem der vorhergehenden Ansprüche, dadurch gekennzeichnet, daß die Zuleitung Besfestigungsmittel zum Festlegen des Herzmuskelinnendrucksensors in dem Herzmuskel aufweist.

9. Herzschrittmachersystem nach einem der vorhergehenden Ansprüche, dadurch gekennzeichnet, daß in der Nähe der Spitze der Zuleitung ein weiterer Sensor angeordnet ist, der zum Erfassen mindestens eines weiteren dem Herzen des Patientien zugeordneten Parameters und zum Erzeugen zusätzlicher Parametersignale dient, die den genannten weiteren Parameter darstellen, daß die Signalverarbeitungseinrichtung für dem Empfang und das Verarbeiten der zusätzlichen Signale geschaltet ist und daß der Regler die Herzschrittmacherfrequenz als Funktion des weiteren Parameters bestimmt.

10. Herzschrittmachersystem nach einem der vorhergehenden Ansprüche, dadurch gekennzeichnet, daß der Regler einen Speicher zum Halten von Algorithmusbefehlen für die Bestimmung der Herzschrittmacherfrequenz als Funktion des Herzmuskelinnendruckes des Patienten aufweist:

11. Herzschrittmachersystem nach Anspruch 10, gekennzeichnet durch eine externe Programmiereinrichtung zur Eingabe von Algorithmusbefehlen in den Regler.

12. Herzschrittmachersystem nach Anspruch 11, dadurch gekennzeichnet, daß die externe Programmiervorrichtung Mittel aufweist, die dazu dienen, in den Regler Daten einzugeben, um diesen zur Steuerung der Herzschrittmacherfrequenz als Funktion mindestens einen anderen Parameters zu steuern.

13. Herzschrittmachersystem nach einem der Ansprüche 1 bis 12, dadurch gekennzeichnet, daß der Regler ein Schwellenwertglied zum Vergleich der Kontraktilitätssignale mit mindestens einem vorherbestimmten Schwellenwert während jedes Zyklus einer Mehrzahl von fortlaufenden Herzschrittmacherzyklen besitzt und daß der Regler die Herzschrittmacherfrequenz als Funktion dieses Vergleiches bestimmt.

## Revendications

1. Un système de stimulation cardiaque adaptable en rythme, comprenant:

un moyen générateur d'impulsions pour produire des impulsions de stimulation,

des moyens de synchronisation (56, 60, 62) pour produire des signaux de synchronisation commandant la durée et le rythme desdites impulsions de stimulation, et connectés pour appliquer lesdits signaux de synchronisation audit moyen générateur d'impulsions afin de commander le rythme des impulsions de stimulation engendrées,

un conducteur (25) pour transmettre lesdites impulsions de stimulation au coeur dudit patient, ledit conducteur (25) étant connecté audit moyen générateur d'impulsions et comportant:

des électrodes (32, 33) pour appliquer lesdites impulsions de stimulation audit coeur du patient,

ledit conducteur comportant également un capteur (26) servant à capter les battements du coeur du patient et à engendrer des signaux cardiaques les représentant,

un capteur de pression positionné sur le conducteur (25) pour une implantation dans le myocarde de manière à capter une pression myocardiaque et à produire un signal la représentant, et

un moyen de traitement de signaux (51) connecté audit conducteur pour recevoir lesdits signaux de pression intramyocardiaque et pour les traiter, et

un dispositif de commande (56) connecté audit moyen de traitement de signaux (51) pour recevoir les signaux de pression intramyocardiaque traités et comportant une sortie reliée audit moyen de synchronisation pour commander le rythme de génération des impulsions de stimulation en concordance avec la pression intramyocardiaque captée,

caractérisé en ce que le dispositif de commande est agencé de manière à répondre pour commander le rythme d'impulsions proportionnellement à une combinaison de la valeur de crête de la pression intramyocardiaque et du taux maximal de variation de la pression intramyocardiaque pendant une contraction, de façon à dériver de la pression intramyocardiaque mesurée une indication de contractivité myocardiaque et en ce qu'il commande le rythme de génération d'impulsions en correspondance avec cette dernière.

2. Le système stimulateur cardiaque tel que revendiqué dans la revendication 1, caractérisé en ce que ledit dispositif de commande comprend un microprocesseur (56) pour recevoir lesdits signaux provenant dudit moyen de traitement de signaux (51) et pour produire un algorithme de détermination dudit rythme en fonction desdits signaux reçus.

3. Un système de stimulation cardiaque tel que revendiqué dans la revendication 2, caractérisé en ce que ledit moyen de traitement de signaux (51) comprend un moyen de conversion analogique-numérique pour convertir lesdits signaux de pression intramyocardiaque en signaux numériques pour une utilisation ultérieure par ledit dispositif de commande.

4. Un système de stimulation cardiaque tel que revendiqué dans une quelconque des revendications précédentes, caractérisé en ce que ledit dispositif de commande (56) est programmable par une source externe (58) de signaux de programmation.

5. Un système de stimulation cardiaque tel que revendiqué dans une quelconque des revendications précédentes, caractérisé en ce que le conducteur (25) comporte une extrémité distale, ledit capteur de pression intramyocardiaque et lesdites électrodes étant disposés à proximité étroite de ladite extrémité distale.

6. Le système de stimulation cardiaque tel que revendiqué dans la revendication 5, caractérisé en ce que le moyen de traitement de signaux (51) peut opérer pour recevoir et traiter lesdits signaux de pression intramyocardiaque sur une base de cycle-à-cycle.

7. Un système de stimulation cardiaque tel que revendiqué dans une quelconque des revendications précédentes, caractérisé en ce que ledit conducteur comprend une extrémité filetée de fixation (35) sur laquelle est monté un capteur de pression intramyocardiaque (42) pour s'engager dans le myocarde du coeur lorsque l'extrémité est vissée dans le myocarde.

8. Le système de stimulation cardiaque tel que revendiqué dans une quelconque des revendications précédentes, caractérisé en ce que ledit conducteur comprend un moyen de fixation pour fixer ledit capteur de pression intramyocardiaque dans le myocarde du coeur.

9. Un système de stimulation cardiaque tel que revendiqué dans une quelconque des revendications précédentes, caractérisé en ce qu'un capteur additionnel est placé à proximité de l'extrémité du conducteur pour capter au moins un paramètre additionnel associé au coeur dudit patient et pour produire des signaux de paramètre additionnel représentatifs dudit paramètre additionnel, ledit moyen de traitement de signaux étant connecté de manière à recevoir et traiter lesdits signaux additionnels, et en ce que ledit dispositif de commande détermine ledit rythme de stimulation en fonction dudit paramètre additionnel.

10. Un système de stimulation cardiaque tel que revendiqué dans une quelconque des revendications précédentes, caractérisé en ce que le dispositif de commande comprend un moyen de mémorisation pour contenir des instructions d'algorithme servant à déterminer un rythme de stimulation en fonction de la pression intramyocardiaque d'un patient.

11. Un système de stimulation cardiaque tel que revendiqué dans la revendication 10, caractérisé par un appareil externe de programmation pour introduire des instructions d'algorithme dans ledit dispositif de commande.

12. Un système de stimulation cardiaque tel que revendiqué dans la revendication 11, caractérisé en ce que l'appareil externe de programmation comporte des moyens pour introduire des données dans le dispositif de commande pour faire en sorte que ce dispositif de commande assure la commande du rythme de stimulation en fonction d'au moins un autre paramètre.

13. Un système de stimulation cardiaque tel que revendiqué dans une quelconque des revendications 1 à 12, caractérisé en ce que le dispositif de commande comporte un moyen à seuil pour comparer lesdits signaux de contractivité avec au moins un seuil prédéterminé pendant chaque cycle d'un ensemble de cycles de stimulation continus et en ce que ledit dispositif de commande détermine un rythme de stimulation en fonction de ladite comparaison.

_**Fig.1A**_

_**Fig.1B**_

_**Fig.1C**_

_**Fig.1D**_

_**Fig. 2A**_

_Fig . 2B_

Fig. 3

## Fig. 4A

_Fig. 4B_

Fig. 4C